# EUROPEAN PATENT APPLICATION

(11) **EP 4 455 175 A1**
(43) Date of publication of application: **30.10.2024**
(21) Application number: 22911925.0
(22) Date of filing: 21.12.2022
(51) Int. Cl.: C08F 230/08, C08F 230/02, C08F 220/18, A61K 8/89, A61K 8/29, A61K 8/02, A61Q 17/04, A61Q 19/00, A61Q 1/00

(54) **POLYMER COMPOUND AND APPLICATION THEREOF**

(30) Priority: 22.12.2021 KR 20210185097
(71) Applicant: Samyang Corporation, Jongno-gu Seoul 03129 (KR)
(72) Inventor: JANG, Seung Hyun, Cheongju-si Chungcheongbuk-do 28608 (KR); KIM, Min Jung, Sejong 30092 (KR); OH, Chun Rim, Seoul 01484 (KR); LEE, Won Jung, Daejeon 34127 (KR); LEE, Chi Wan, Daejeon 34127 (KR)
(74) Representative: Schrell, Andreas
(86) International application number: PCT/KR2022/020960
(87) International publication number: WO 2023/121292

(57) **Abstract**

The present invention relates to: a polymer compound capable of modifying the surface of an inorganic nanopowder through chemical bonding to the surface; and an application thereof, and, more specifically, to a polymer compound and an application thereof, the polymer compound readily and rapidly chemically bonding to the surface of an inorganic nanoparticle powder so as to modify the surface of the inorganic nanoparticle and form a polymer coating layer, and being economically and efficiently prepared.

## Description

### TECHNICAL FIELD

The present invention relates to a polymer compound capable of modifying the surface of inorganic nano powder through chemical bonding to the surface and application thereof, and more specifically, a polymer compound which readily and rapidly reacts with and chemically bonds to the surface of inorganic nanoparticle powder, and thus can modify the surface of the inorganic nanoparticle powder and form a polymer coating layer, and also can be prepared economically and efficiently, and application thereof.

### BACKGROUND ART

Inorganic nanoparticle powder generally used in cosmetic industry is mainly titanium dioxide (TiO₂) or zinc oxide (ZnO). These inorganic nanoparticle powders are used mainly as pigments in tone-up cream formulation that brightens skin tone or inorganic sunscreens in formulation of sunscreen products. In particular, recently global warming caused by ozone layer destruction and skin exposure to ultraviolet rays are rapidly increasing. In order to protect skin from the increasing ultraviolet rays, the market for sunscreen products is growing rapidly by more than 10% every year. UV-blocking products use a dispersion formulation system based on inorganic nanoparticles and organic UV absorber to protect skin from UV rays. Inh addition, such UV-blocking products must have effective UV-blocking capabilities and at the same time have light feel, smooth application, and clean finish. However, although inorganic nanoparticle powder with high density and strong inter-particle interaction is a key material with ultraviolet scattering ability, it has a negative effect on all other performances. Accordingly, attempts are being made to improve skin stability and dispersibility and enhance product performance through modification of the surface properties of inorganic nanoparticle powder. There are efforts to improve dispersion instability through organic coating or silica coating, but the fundamental problem is not solved. In order to improve the essential properties of inorganic materials, new coating technologies based on organic materials are needed, but coating technologies that satisfy both productivity and product performance are undeveloped.

For the surface coating of inorganic nano powder (TiO₂, ZnO) used in conventional cosmetic formulations used generally, approaches using materials such as fatty acids, silicone, amino acids, fluorine compounds, etc. have been attempted, but these materials have some problems in use, such as the following. In case of fatty acid coating, oil resistance to sebum is reduced, causing shine and discoloration, and in case of silicone coating, it feels dry and rough. In case of amino acid coating, water/oil resistance is reduced and a heavy feeling occurs, and in case of fluorine compound coating, the make-up appears stuffy or unnatural, resulting in reduced formability. In order to improve such disadvantage of low skin compatibility, attempts are being made to develop new inorganic nano powder coating technology.

In particular, a technology to uniformly form a thin layer of biocompatible molecules on the surface of inorganic nano powder for increasing the preferred feeling of use such as skin adhesion, moisturizing and adhesion, etc. has been reported (for example, Korean Patent No. 10-1767207; Polymer, 54 (21), 2013, 5609-5614). However, the method of polymerizing molecules for inorganic nano powder surface coating used in the above technology is surface growth polymerization and surface deposition polymerization, which limits the strengthening of surface thin film thickness of the inorganic nano powder, requires a surface initiator linker, and not only the polymerization efficiency is low, but also the introduction of hydrophobic monomers into the polymer structure for improving skin adhesion is limited. In addition, the production requires expensive multi-step process which presents various disadvantages such as reduced productivity, making development difficult.

### CONTENTS OF THE INVENTION

### PROBLEMS TO BE SOLVED

The purpose of the present invention is to provide a polymer compound which readily and rapidly reacts with and chemically bonds to the surface of inorganic nanoparticle powder used in cosmetic formulation, and thus can modify the surface of the inorganic nanoparticle powder and form a polymer coating layer, and also can be prepared economically and efficiently, and application thereof.

### TECHNICAL MEANS

In order to achieve the above-stated purpose, the present invention provides a polymer compound having a structure represented by the following formula 1: 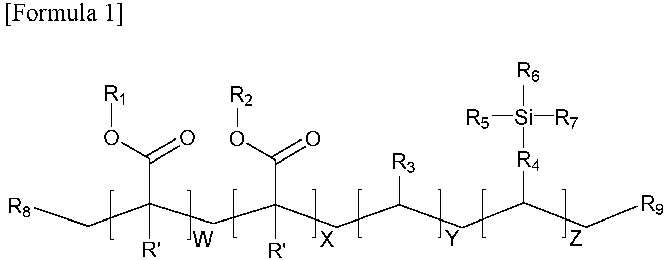 wherein
R' is the same or different, and each independently represents hydrogen or methyl group,
R₁ is a group containing phosphorylcholine-like phospholipid structure,
R₂ is independently hydrogen; alkoxy group; amino group; epoxy group; or C1-C20 aliphatic hydrocarbon group or C6-C20 aromatic hydrocarbon group unsubstituted or substituted with one or more functional groups,
R₃ is independently C1-C20 aliphatic hydrocarbon group or C6-C20 aromatic hydrocarbon group unsubstituted or substituted with alkyl group, alkoxy group, epoxy group, hydroxy group, or acid functional group,
R₄ is C1-C10 aliphatic hydrocarbon group,
R₅, R₆ and R₇ are the same or different and are each independently hydrogen; or C1-C20 aliphatic hydrocarbon group or C6-C20 aromatic hydrocarbon group unsubstituted or substituted with alkyl group, alkoxy group, epoxy group, hydroxy group or acid functional group,
R₈ and R₉ are the same or different and are each independently hydrogen; or a group containing hydroxy, carboxylic acid or alkoxy functional group, provided that at least one of R₈ and R₉ contains hydroxy, carboxylic acid or alkoxy functional group,
W, X, Y and Z are the mole fractions of the corresponding repeating units and are each independently 0 to 1, provided that at least one of W and X is greater than 0, and W+X+Y+Z=1.

In other aspect, the present invention provides an inorganic nano powder having a surface to which the polymer compound of the present invention is chemically bonded.

In another aspect, the present invention provides a powder formulation comprising the inorganic nano powder of the present invention.

### EFFECT OF THE INVENTION

The polymer compound of the present invention readily and rapidly reacts with and chemically bonds to the surface of inorganic nanoparticle powder used in cosmetic formulation, and thus can modify the surface of the inorganic nanoparticle powder and form a polymer coating layer, and the polymer production process is very economical since it requires neither expensive process nor complicated multi-step production processes, contributing to improved productivity.

### BRIEF EXPLANATION OF THE DRAWINGS

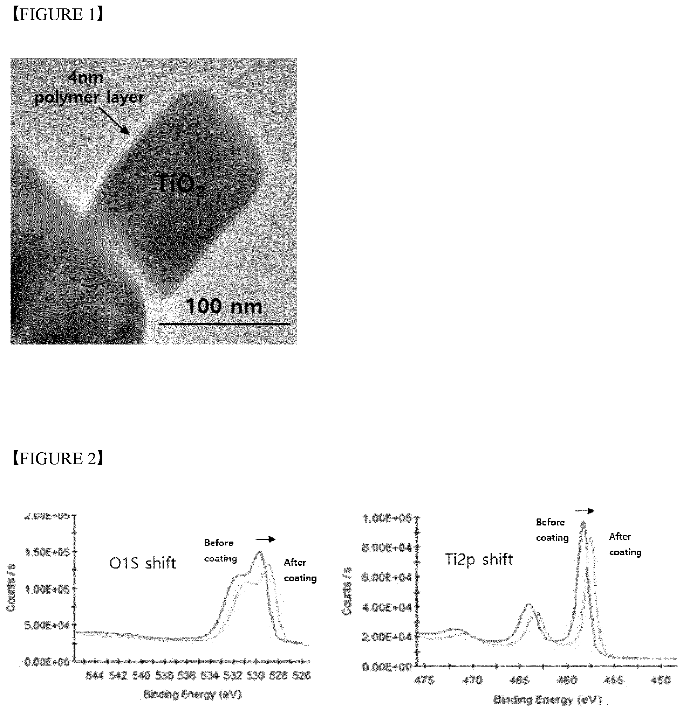

Figure 1 is HR-TEM image of the inorganic nano powder prepared in Coating Example 21 of the present invention, showing that a polymer layer is formed on the TiO₂ surface coated using Polymer Compound 1.
Figure 2 is XPS analysis results of the inorganic nano powder prepared in Coating Example 21 of the present invention, showing the difference in binding energy (O1S, Ti2p) values of TiO₂ surface before and after coating and proving that the chemical bond between the polymer compound and the TiO₂ surface is well established.

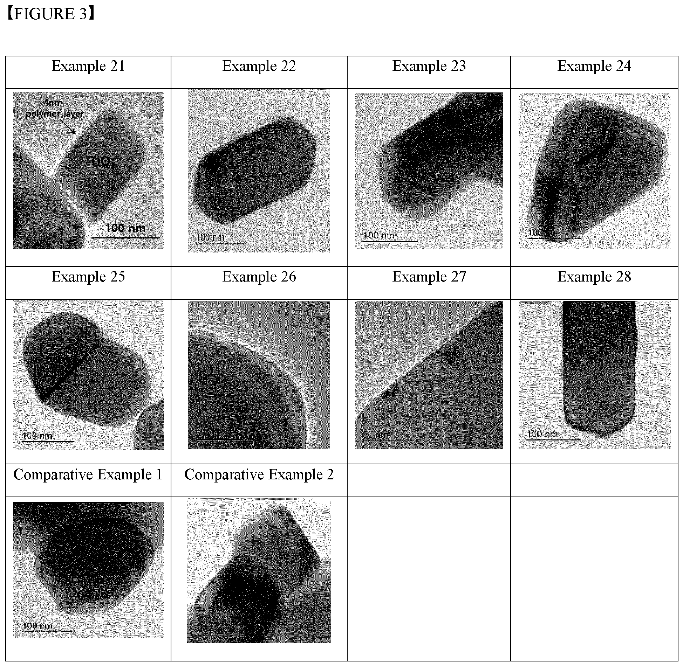

Figure 3 is HR-TEM images of inorganic nano powders prepared in Coating Examples 21 to 28 of the present invention and Comparative Examples 1 and 2, respectively.

### CONCRETE MODE FOR CARRYING OUT THE INVENTION

The present invention will be explained in detail below.

The polymer compound of the present invention has a structure represented by the following formula 1: 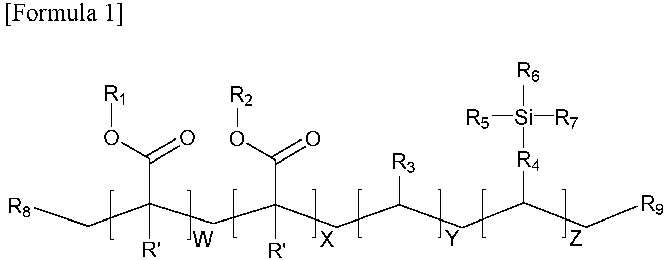 wherein
R' is the same or different, and each independently represents hydrogen or methyl group,
R₁ is a group containing phosphorylcholine-like phospholipid structure,
R₂ is independently hydrogen; alkoxy group; amino group; epoxy group; or C1-C20 aliphatic hydrocarbon group or C6-C20 aromatic hydrocarbon group unsubstituted or substituted with one or more functional groups,
R₃ is independently C1-C20 aliphatic hydrocarbon group or C6-C20 aromatic hydrocarbon group unsubstituted or substituted with alkyl group, alkoxy group, epoxy group, hydroxy group, or acid functional group,
R₄ is C1-C10 aliphatic hydrocarbon group,
R₅, R₆ and R₇ are the same or different and are each independently hydrogen; or C1-C20 aliphatic hydrocarbon group or C6-C20 aromatic hydrocarbon group unsubstituted or substituted with alkyl group, alkoxy group, epoxy group, hydroxy group or acid functional group,
R₈ and R₉ are the same or different and are each independently hydrogen; or a group containing hydroxy, carboxylic acid or alkoxy functional group, provided that at least one of R₈ and R₉ contains hydroxy, carboxylic acid or alkoxy functional group,
W, X, Y and Z are the mole fractions of the corresponding repeating units and are each independently 0 to 1, provided that at least one of W and X is greater than 0, and W+X+Y+Z=1.

In an embodiment, in the above formula 1, R₁ may be a group containing phosphorylcholine-like phospholipid structure represented by the following formula (2): 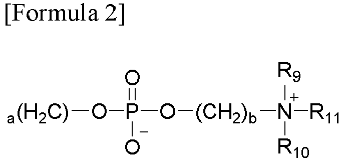 wherein
a is an integer of 0 to 2,
b is an integer of 2 to 4,
R₁₀, R₁₁ and R₁₂ are the same or different, and each independently represents hydrogen, C1-C6 hydrocarbon group, or -(CH₂)ₙ-OH group, where n is an integer of 1 to 6.

In an embodiment, in the above formula 1, the alkyl group may be C1-C20 alkyl group or C3-C20 cycloalkyl group, the alkoxy group may be C1-C20 alkoxy group or C3-C20 cycloalkoxy group, the acid functional group may be carboxylic acid group, and the aliphatic hydrocarbon group may be saturated or unsaturated aliphatic hydrocarbon group.

In an embodiment, in the above formula 1, the functional group substituted for the C1-C20 aliphatic hydrocarbon group or the C6-C20 aromatic hydrocarbon group may be alkoxy group, aryloxy group, ether group, halogen group, isocyanate group, lactone group, hydroxy group, or acid functional group.

In the above formula 1, W is 0 to 1, and more concretely, it may be 0.01 or more, 0.05 or more, or 0.1 or more, and also may be 1 or less, 0.99 or less, 0.95 or less, 0.9 or less, 0.85 or less, or 0.8 or less, but it is not limited thereto.

In the above formula 1, X is 0 to 1, and more concretely, it may be 0.01 or more, 0.05 or more, or 0.1 or more, and also may be 1 or less, 0.9 or less, 0.8 or less, 0.7 or less, 0.6 or less, or 0.5 or less, but it is not limited thereto.

In the above formula 1, Y is 0 to 1, and more concretely, it may be 0.01 or more, 0.05 or more, or 0.1 or more, and also may be 1 or less, 0.95 or less, 0.9 or less, 0.85 or less, or 0.8 or less, but it is not limited thereto.

In the above formula 1, Z is 0 to 1, and more concretely, it may be 0.01 or more, 0.02 or more, or 0.05 or more, and also may be 1 or less, 0.9 or less, 0.8 or less, 0.7 or less, 0.6 or less, or 0.5 or less, but it is not limited thereto.

In an embodiment, in the above formula 1, the unsaturated phospholipid monomer providing the repeat unit represented by the mole fraction W may be one or more selected from the group consisting of, for example, 2-((meth)acryloyloxy)ethyl-2'-(trimethylammonio)ethyl phosphate, 3-((meth)acryloyloxy)propyl-2'-(trimethylammonio)ethyl phosphate, 4-((meth)acryloyloxy)butyl-2'-(trimethylammonio)ethyl phosphate, 5-((meth)acryloyloxy)pentyl-2'-(trimethylammonio)ethyl phosphate, 6-((meth)acryloyloxy)hexyl-2'-(trimethylammonio)ethyl phosphate, 2-((meth)acryloyloxy)ethyl-2'-(triethylammonio)ethyl phosphate, 2-((meth)acryloyloxy)ethyl-2'-(tripropylammonio)ethyl phosphate, 2-((meth)acryloyloxy)ethyl-2'-(tributylammonio)ethyl phosphate, 2-((meth)acryloyloxy)propyl-2'-(trimethylammonio)ethyl phosphate, 2-((meth)acryloyloxy)butyl-2'-(trimethylammonio)ethyl phosphate, 2-((meth)acryloyloxy)pentyl-2'-(trimethylammonio)ethyl phosphate, 2-((meth)acryloyloxy)hexyl-2'-(trimethylammonio)ethyl phosphate, 2-(vinyloxy)ethyl-2'-(trimethylammonio)ethyl phosphate, 2-(allyloxy)ethyl-2'-(trimethylammonio)ethyl phosphate, 2-(p-vinylbenzyloxy)ethyl-2'-(trimethylammonio)ethyl phosphate, 2-(p-vinylbenzoyloxy)ethyl-2' -(trimethylammonio)ethyl phosphate, 2-(styryloxy)ethyl-2'-(trimethylammonio)ethyl phosphate, 2-(p-vinylbenzyl)ethyl-2'-(trimethylammonio)ethyl phosphate, 2-(vinyloxycarbonyl)ethyl-2'-(trimethyl ammonio)ethylphosphate, 2-(allyloxycarbonyl)ethyl-2'-(trimethylammonio)ethyl phosphate, 2-(acryloylamino)ethyl-2'-(trimethylammonio)ethyl phosphate, 2-(vinylcarbonylamino)ethyl-2'-(trimethylammonio)ethyl phosphate, 2-(allyloxycarbonylamino)ethyl-2'-(trimethylammonio)ethyl phosphate, 2-(buteroyloxy)ethyl-2'-(trimethylammonio)ethyl phosphate, 2-(crotonoyloxy)ethyl-2' -(trimethylammonio)ethyl phosphate, ethyl-(2' - trimethylammonioethylphosphorylethyl)fumarate, butyl-(2'-trimethylammonioethylphosphorylethyl)fumarate, hydroxyethyl-(2'-trimethylammonioethylphosphorylethyl)fumarate, ethyl-(2'-trimethylammonioethylphosphorylethyl)fumarate, butyl-(2'-trimethylammonioethylphosphorylethyl)fumarate, and hydroxyethyl-(2'-trimethylammonioethylphosphorylethyl)fumarate.

In an embodiment, in the above formula 1, R₂ may be independently selected from the group consisting of, for example, hydrogen, methyl, ethyl, propyl, butyl, pentyl, hexyl, cyclohexyl, heptyl, octyl, nonyl, decyl, lauryl, tetradecyl, hexadecyl, isobornyl, dicyclopentanyl, dicyclopentenyl, benzyl, 2-methoxyethyl, 2-ethoxyethyl, succinyl, monoalkyl succinate, monoalkyl fumarate, glycidyl, 3,4-epoxybutyl, 2,3-epoxycyclohexyl, 3,4-epoxycyclohexylmethyl , 3-methyloxetane-3-methyl, 3-ethyloxetane-3-methyl, NH₂, NHCH₃, allyl, 2-hydroxyethyl, 3-oxypropyl methyldimethoxysilane, tetrahydrofurfuryl, ethylene glycol dicyclopentenyl ether, 2-ethyl-2-methyl-1,3-dioxoreynylmethyl, 2-isocyanatoethyl, 4-morpholine, phenylglycidylether, phenoxydiethyleneglycol, methoxypolyethyleneglycol, 2-[4-(1-methyl-1-phenylethyl)-phenoxy]ethyl, t-butyl, 2-oxypropyl hexahydrophthalate, gamma-butyrolactone, propoxyethyl, n-isobutoxymethyl, 2-oxy ethylphthalate, 1,1,1,3,3,3-hexafluoroisopropyl, octafluoropentyl, tetrafluoropropyl, heptadecafluorodecyl, and tribromophenyl.

In an embodiment, in the above formula 1, R₃ may be independently selected from the group consisting of, for example, phenyl, p-methylphenyl, and p-acetoxyphenyl.

In an embodiment, in the above formula 1, R₄ may be, for example, methylene group or ethylene group.

In an embodiment, in the above formula 1, R₅, R₆ and R₇ may be each independently selected from the group consisting of, for example, hydrogen, methyl, ethyl, n-propyl, isopropyl, n-butyl, tert-butyl, phenyl, naphthyl, vinyl, methacryloxymethyl, 2-methacryloxyethyl, 3-methacryloxypropyl, acryloxymethyl, 2-acryloxyethyl, 3-acryloxypropyl, 3-glycidyloxypropyl, 2-epoxycyclohexylethyl, 3-epoxycyclohexylpropyl, and O-R₁₂ (where R₁₂ is hydrogen, methyl, ethyl, or propyl).

The polymer compound of the present invention can be prepared by any one of several commonly known polymerization methods such as radical polymerization, cationic polymerization, anionic polymerization, condensation polymerization, etc. and in terms of the easiness and economics of preparation of polymer compounds, it is preferable to use radical polymerization. In addition, it can be prepared by mixing monomers with a polymerization solvent, heating the mixture to an appropriate temperature according to the half-life temperature of the thermal initiator used, and then removing oxygen under nitrogen atmosphere.

As conditions for polymerization of polymer compound, polymerization time and temperature may vary depending on the type of thermal initiator used. As a radical polymerization initiator, a generally known thermal initiator can be used, and more concretely, 2,2'-azobisisobutyronitrile, 2,2'-azobis-2,4-dimethyl valeronitrile, 2,2'-azobis-(4-methoxy-2,4-dimethylvaleronitrile), benzoyl peroxide, lauroyl peroxide, t-butyl peroxypivalate, 1,1'-bis-(bis-t-butylperoxy)cyclohexane, 1,1'-azobis(cyclohexane-1-carbonitrile), 4,4'-azobis(4-cyanovaleric acid), dimethyl-2,2'-azobis(2-methylpropionate), 2,2'-azobis[2-methyl-N-(2-hydroxyethyl)propionamide, 2,2'-azobis(N-butyl-2-methylpropionamide, 2,2' -azobis[2-(2-imidazolinyl)propane]dihydrochloride, 2,2'-azobis[2-(2-imidazolinyl)propane], 2,2'-azobis[2-methyl-N-(2-hydroxyethyl)propionamide], 4,4'-azobis(4-cyanovaleric acid), 2,2'-azobis[N-(2-carboxyethyl)-2-methylpropionamidine]tetrahydrite, dimethyl-2,2'-azobis(2-methylpropionate), 2,2'-azobis(4-methoxy-2,4-dimethylvaleronitrile), etc. may be used alone or in combination of two or more.

In an embodiment, when preparing the polymer compound, generally known chain transfer agent can be used to control the weight average molecular weight (Mw) of the polymer, and more concretely, 1-dodecanethiol, octadecanethiol, pentaerythol tetrakis(3-mercaptobutyrate), 1,3,5-tris(3-mercaptobutyroethyl)-1,3,5-triazine -2,4,6-trione, thioglycolic acid, 3-mercaptopropyl trimethoxysilane, 3-mercaptopropyl triethoxysilane, 3-mercaptopropyl dimethoxysilane, 3-mercaptopropyl diethoxysilane, 2-mercaptopropionic acid, 3-mercaptopropionic acid, etc. may be used.

In an embodiment, the weight average molecular weight (Mw, g/mol) of the polymer compound of the present invention may be 1,000 to 200,000 g/mol, and the polydispersity index (PDI) may be 1.0 to 20, and more concretely, the weight average molecular weight (Mw) may be 5,000 to 100,000 g/mol, the polydispersity index (PDI) may be 1.0 to 10, and even more concretely, the weight average molecular weight (Mw) may be 10,000 to 50,000 g/mol, and the polydispersity index (PDI) may be 1.0 to 5.0, but it is not limited thereto. In addition, the polymer compound of the present invention may be a homopolymer or a random copolymer that is not restricted to the arrangement order of each polymer unit, but it is not limited thereto.

In consideration of usability with monomer, initiator, chain transfer agent, and other additive, an appropriate solvent can be used in the polymerization of a polymer compound. For example, the solvent may be alcohol such as methanol, ethanol, etc.; ether such as dichloroethyl ether, n-butyl ether, diisoamyl ether, methylphenyl ether, tetrahydrofuran, etc.; glycol ether such as ethylene glycol monomethyl ether, ethylene glycol monoethyl ether, etc.; cellosolve acetate such as methyl cellosolve acetate, ethyl cellosolve acetate, diethyl cellosolve acetate, etc.; carbitol such as methyl ethyl carbitol, diethyl carbitol, diethylene glycol monomethyl ether, diethylene glycol monoethyl ether, diethylene glycol dimethyl ether, diethylene glycol methyl ethyl ether, diethylene glycol diethyl ether, etc.; propylene glycol alkyl ether acetate such as propylene glycol methyl ether acetate, propylene glycol propyl ether acetate, etc.; aromatic hydrocarbon such as toluene, xylene, etc.; ketone such as methyl ethyl ketone, cyclohexanone, 4-hydroxy-4-methyl-2-pentanone, methyl-n-propyl ketone, methyl-n-butyl ketone, methyl-n-amyl ketone, 2-heptanone, etc.; saturated aliphatic monocarboxylic acid alkyl ester such as ethyl acetate, n-butyl acetate, isobutyl acetate, etc.; lactic acid esters such as methyl lactate, ethyl lactate, etc.; oxyacetic acid alkyl ester such as methyl oxyacetate, ethyl oxyacetate, butyl oxyacetate, etc.; alkoxy acetate alkyl ester such as methoxy methyl acetate, methoxy ethyl acetate, methoxy butyl acetate, ethoxy methyl acetate, ethoxy ethyl acetate, etc.; 3-oxypropionic acid alkyl ester such as methyl 3-oxypropionate, ethyl 3-oxypropionate, etc.; 3-alkoxy propionic acid alkyl ester such as 3-methoxy methyl propionate, 3-methoxy ethyl propionate, 3-ethoxy ethyl propionate, 3-ethoxy methyl propionate, etc.; 2-oxypropionic acid alkyl ester such as methyl 2-oxypropionate, ethyl 2-oxypropionate, propyl 2-oxypropionate, etc.; 2-alkoxy propionic acid alkyl ester such as 2-methoxy methyl propionate, 2-methoxy ethyl propionate, 2-ethoxy ethyl propionate, 2-ethoxy methyl propionate, etc.; 2-oxy-2-methylpropionate ester such as methyl 2-oxy-2-methyl propionate, ethyl 2-oxy-2-methyl propionate, etc.; monooxy monocarboxylic acid alkyl ester of alkyl 2-alkoxy-2-methyl propionate such as methyl 2-methoxy-2-methyl propionate, ethyl 2-ethoxy-2-methyl propionate, etc.; ester such as ethyl 2-hydroxy propionate, ethyl 2-hydroxy-2-methyl propionate, ethyl hydroxy acetate, and methyl 2-hydroxy-3-methyl butanoate, etc.; ketonic acid ester such as ethyl pyruvate, etc.; and it is also possible to use a single solvent selected from, or a mixture solvent of two or more selected from, N-methylformamide, N,N-dimethylformamide, N-methylformanilide, N-methylacetamide, N,N-dimethylacetamide, N-methylpyrrolidone, dimethylsulfoxide, benzyl ethyl ether, dihexyl ether, acetyl acetone, isophorone, caproic acid, caprylic acid, 1-octanol, 1-nonanol, benzyl alcohol, benzyl acetate, ethyl benzoate, diethyl oxalate, diethyl maleate, γ-butyrolactone, ethylene carbonate, propylene carbonate, phenyl cellosolve acetate and the like.

The polymer compound of the present invention readily and rapidly reacts with and chemically bonds to the surface of inorganic nanoparticle powder used in cosmetic formulation, and thus can modify the surface of the inorganic nanoparticle powder and form a polymer coating layer.

Therefore, in other aspect, the present invention provides an inorganic nano powder having a surface to which the polymer compound of the present invention is chemically bonded.

In an embodiment, the inorganic nano powder may be an inorganic nano material having a coating layer of the polymer compound of the present invention on its surface (for example, titanium dioxide, zinc oxide, mica, sericite, kaolin, silicic anhydride, boron nitride, or talc with an average particle size of 100 nm or less).

In another aspect, the present invention provides a powder formulation comprising the inorganic nano powder of the present invention.

In an embodiment, the powder formulation may be UV blocking powder, cosmetic powder, or color powder, but it is not limited thereto.

The present invention will be explained below in more detail with reference to the following Examples and Comparative Examples. However, the scope of the present invention is not limited thereby in any manner.

### EXAMPLES

### Examples 1 to 20

### <Polymer compound preparation>

Polymerization was carried out using monomers A to E of the types and ratios shown in Table 1 below, and the thermal initiator and chain transfer agent of the types and amounts (parts by weight based on total 100 parts by weight of the monomers) shown in Table 1 below. As polymerization conditions, a three-neck round bottom flask was used, anhydrous ethanol was used as a polymerization solvent, and the mixture was dissolved to have a solid content of total 35 parts by weight, and then polymerized by applying temperature. After polymerization was completed, 100 ppm of t-butylhydroquinone (t-BHQ) was added as a polymerization inhibitor to prevent further polymerization. The weight average molecular weight (Mw) and polydispersity index (PDI) upon completion of polymerization are shown in Table 1 below. At this time, the weight average molecular weight (Mw) and polydispersity index (PDI) were managed using a chain transfer agent.

### [Components used]

A: 2-methacryloyloxyethyl phosphorylcholine
B: 3-trimethoxypropyl methacrylate
C: tert-butyl methacylate
D: methyl methacrylate
E: allyl triethoxysilane
F: 2,2'-azobisisobutyronitrile
G: 4,4'-azobis(4-cyanovaleric acid)
H: 3-mercaptopropyltrimethoxysilane
I: 1-dodecanethiol

**[Table 1]**

| Example | Monomers for polymerization (mol%) | | | | | Total | Initiator (parts by weight) | | Chain transfer agent (parts by weight) | | GPC | | Compound No. |
|---|---|---|---|---|---|---|---|---|---|---|---|---|---|
| | A | B | C | D | E | | F | G | H | I | Mw | PDI | |
| 1 | 50 | 20 | 15 | 10 | 5 | 100 | 1.0 | 3.0 | 2.0 | - | 24,232 | 4.3 | 1 |
| 2 | 80 | 20 | 0 | 0 | 0 | 100 | - | 3.0 | 1.0 | 1.0 | 46,231 | 5.5 | 2 |
| 3 | 80 | 0 | 20 | 0 | 0 | 100 | 0.5 | 1.5 | 1.0 | 0.5 | 37,816 | 4.7 | 3 |
| 4 | 60 | 10 | 20 | 5 | 5 | 100 | 1.0 | 3.0 | 0.5 | - | 58,556 | 4,0 | 4 |
| 5 | 10 | 50 | 20 | 20 | 0 | 100 | - | 4.0 | 1.0 | 0.5 | 60,362 | 3.2 | 5 |
| 6 | 100 | 0 | 0 | 0 | 0 | 100 | 2.0 | 2.0 | 1.0 | 1.5 | 25,943 | 4.6 | 6 |
| 7 | 80 | 0 | 20 | 0 | 0 | 100 | 2.5 | - | 1.0 | 0.5 | 32,215 | 4.2 | 7 |
| 8 | 50 | 30 | 0 | 10 | 10 | 100 | 2.5 | 0.5 | - | 2.0 | 37,426 | 3.6 | 8 |
| 9 | 30 | 25 | 20 | 5 | 20 | 100 | 3.0 | 1.0 | - | 1.5 | 29,843 | 4. | 9 |
| 10 | 10 | 30 | 20 | 40 | 0 | 100 | 2.0 | 1.5 | 2.5 | - | 45,345 | 3.4 | 10 |
| 11 | 60 | 0 | 25 | 10 | 5 | 100 | 1.0 | 1.0 | - | 1.0 | 53,076 | 4.3 | 11 |
| 12 | 50 | 25 | 0 | 25 | 0 | 100 | 4.0 | - | 3.0 | - | 27,843 | 3.6 | 12 |
| 13 | 50 | 25 | 0 | 0 | 25 | 100 | 2.0 | 1.5 | 1.0 | 0.5 | 44,235 | 4.8 | 13 |
| 14 | 10 | 40 | 30 | 10 | 10 | 100 | 1.0 | 1.0 | 2.0 | - | 40,674 | 3.9 | 14 |
| 15 | 0 | 20 | 50 | 30 | 0 | 100 | 0.5 | 1.5 | - | 1.0 | 33,238 | 3.6 | 15 |
| 16 | 10 | 0 | 15 | 35 | 40 | 100 | 2.5 | - | - | 1.5 | 36,842 | 4.2 | 16 |
| 17 | 10 | 30 | 15 | 0 | 45 | 100 | 2.5 | - | - | 1.5 | 32,760 | 4.5 | 17 |
| 18 | 50 | 0 | 50 | 0 | 0 | 100 | 3.5 | - | 2.5 | - | 41,838 | 3.7 | 18 |
| 19 | 10 | 20 | 30 | 40 | 0 | 100 | 2.0 | - | 2.0 | 1.0 | 31,522 | 4.6 | 19 |
| 20 | 10 | 40 | 30 | 20 | 0 | 100 | 2.0 | 2.0 | - | 2.0 | 21,532 | 2.4 | 20 |

### Examples 21 to 28

### <Evaluation of TiO₂ coating applied with Polymer Compound>

Coating was carried out on the surface of titanium dioxide using each of Polymer Compounds 1, 2, 6, 7, 9, 12, 15, and 17 of Table 1 prepared as above under the conditions shown in Table 2 below. Commercial titanium dioxide was purchased and used. After coating, HR-TEM image analysis was conducted to confirm whether a polymer compound layer was formed on the surface of titanium dioxide, and XPS analysis was conducted to confirm the shift in binding energy value. The results are shown in Table 2 below.

### Comparative Examples 1 and 2

Coating was carried out on the surface of titanium dioxide using each of the polymer compounds of the following formulas 3 and 4, and the results were evaluated in the same manner and are shown in Table 2 below.

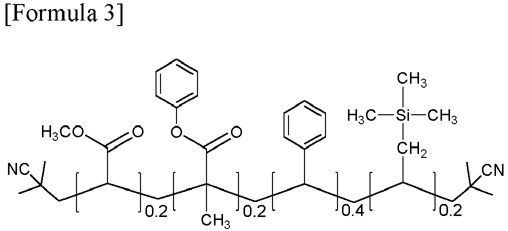

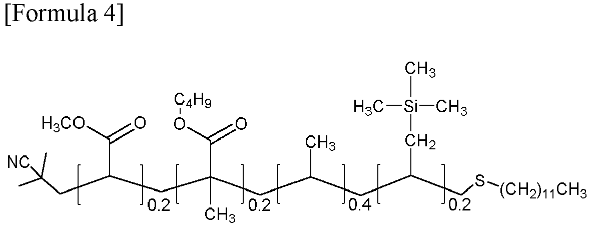

### TiO₂ surface coating and washing test

The TiO₂ surface coating and washing process were carried out as follows. First, in the coating process, 97g of TiO₂ was added to a beaker, 100g of anhydrous ethanol solvent was added, and homogenization was done by stirring at room temperature at high speed with a homodisperse, and then 3g of the polymer compound was added four times while stirring with a homodisperse. Homogenization was done by stirring for 10 minutes at room temperature, and the product was dried on aluminum foil at 100°C for 12 hours, pulverized with a hammer mill, and filtered through a 60-mesh mesh. After the coating process was completed, in the washing process, 30g of coated TiO₂ was added to 120g of ionized water in a 200 ml PET sample bottle, stirred at high speed for 1 hour, and then centrifuged (3000 rpm, 10 min) to precipitate the inorganic powder. Then, the clear upper layer solution was removed, and the solid precipitate in the lower layer was transferred to aluminum foil and dried at 100°C for 12 hours, and the dried TiO₂ powder was finely ground in a bowl and stored.

### Confirmation of polymer compound coating layer formation on the TiO₂ surface (TEM image observation)

In order to confirm whether the coating of the polymer compound proceeded well on the surface of the inorganic nano powder (TiO₂) that had undergone the coating and washing processes, measurements were made using HR-TEM equipment. Meanwhile, cases where a polymer layer was formed and observed in the TEM image even after a strong washing process are indicated as "O" whereas cases where the polymer layer was not formed but washed away and no polymer layer was observed are indicated as "X."

### Confirmation of the presence or absence of chemical bonding in the polymer compounds on the TiO₂ surface (XPS analysis confirmation)

It was confirmed through XPS analysis whether the prepared polymer compound was chemically well bonded to the TiO₂ surface through a chemical reaction. X-ray photoelectron spectroscopy (XPS) is one of the most widely used analytical techniques in surface analysis. Since the binding energy measured using XPS is the unique energy of the element, the element in the analysis sample can be analyzed, and since the binding energy changes depending on the chemical bond state, information about the chemical bond state can be obtained. The binding energy of the O1S level and the binding energy of the Ti2p level of TiO₂ were confirmed. Changes in surface modification through chemical bonding are expressed as a shift in the binding energy value of each level, through which the presence or absence of surface coating can be confirmed. In the case of the binding energy value, if it appears above 0.3 eV, it is considered a meaningful shift. Cases where a shift in the binding energy value was observed are indicated as "O" whereas cases where no shift was observed are indicated as "X."

**[Table 2]**

| | | Coating conditions | | | | | Coating results | | |
|---|---|---|---|---|---|---|---|---|---|
| | | Polymer Compound No. | Polymer amount (g) | TiO₂ amount (g) | Solvent (g) | Coating time (min) | Coating thickness (nm) | Polymer compound coating layer formation (TEM image) | Binding energy shift after coating (XPS, OS1, Ti2p) |
| Examples | 21 | 1 | 3 | 97 | 100 | 10 | 4.0 | O | O |
| | 22 | 2 | 3 | 97 | 100 | 10 | 5.6 | O | O |
| | 23 | 6 | 3 | 97 | 100 | 10 | 6.4 | O | O |
| | 24 | 7 | 3 | 97 | 100 | 10 | 5.2 | O | O |
| | 25 | 9 | 3 | 97 | 100 | 10 | 4.5 | O | O |
| | 26 | 12 | 3 | 97 | 100 | 10 | 6.6 | O | O |
| | 27 | 15 | 3 | 97 | 100 | 10 | 4.6 | O | O |
| | 28 | 17 | 3 | 97 | 100 | 10 | 5.7 | O | O |
| Comparative Examples | 1 | Formula 3 | 3 | 97 | 100 | 10 | Not observed | X | X |
| | 2 | Formula 4 | 3 | 97 | 100 | 10 | Not observed | X | X |

## Claims

1. A polymer compound having a structure represented by the following formula 1: 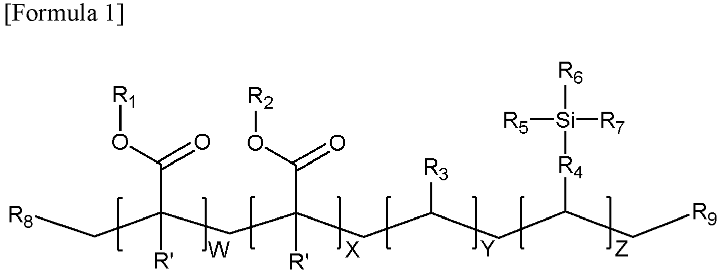 wherein
R' is the same or different, and each independently represents hydrogen or methyl group,
R₁ is a group containing phosphorylcholine-like phospholipid structure,
R₂ is independently hydrogen; alkoxy group; amino group; epoxy group; or C1-C20 aliphatic hydrocarbon group or C6-C20 aromatic hydrocarbon group unsubstituted or substituted with one or more functional groups,
R₃ is independently C1-C20 aliphatic hydrocarbon group or C6-C20 aromatic hydrocarbon group unsubstituted or substituted with alkyl group, alkoxy group, epoxy group, hydroxy group, or acid functional group,
R₄ is C1-C10 aliphatic hydrocarbon group,
R₅, R₆ and R₇ are the same or different and are each independently hydrogen; or C1-C20 aliphatic hydrocarbon group or C6-C20 aromatic hydrocarbon group unsubstituted or substituted with alkyl group, alkoxy group, epoxy group, hydroxy group or acid functional group,
R₈ and R₉ are the same or different and are each independently hydrogen; or a group containing hydroxy, carboxylic acid or alkoxy functional group, provided that at least one of R₈ and R₉ contains hydroxy, carboxylic acid or alkoxy functional group,
W, X, Y and Z are the mole fractions of the corresponding repeating units and are each independently 0 to 1, provided that at least one of W and X is greater than 0, and W+X+Y+Z=1.

2. The polymer compound of Claim 1, wherein R₁ is a group containing phosphorylcholine-like phospholipid structure represented by the following formula (2): 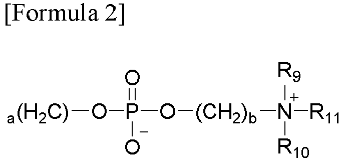 wherein
a is an integer of 0 to 2,
b is an integer of 2 to 4,
R₁₀, R₁₁ and R₁₂ are the same or different, and each independently represents hydrogen, C1-C6 hydrocarbon group, or -(CH₂)ₙ-OH group, where n is an integer of 1 to 6.

3. The polymer compound of Claim 1, wherein the unsaturated phospholipid monomer providing the repeat unit represented by the mole fraction W is one or more selected from the group consisting of:
2-((meth)acryloyloxy)ethyl-2'-(trimethylammonio)ethyl phosphate,
3-((meth)acryloyloxy)propyl-2'-(trimethylammonio)ethyl phosphate,
4-((meth)acryloyloxy)butyl-2'-(trimethylammonio)ethyl phosphate,
5-((meth)acryloyloxy)pentyl-2'-(trimethylammonio)ethyl phosphate,
6-((meth)acryloyloxy)hexyl-2'-(trimethylammonio)ethyl phosphate,
2-((meth)acryloyloxy)ethyl-2'-(triethylammonio)ethyl phosphate,
2-((meth)acryloyloxy)ethyl-2'-(tripropylammonio)ethyl phosphate,
2-((meth)acryloyloxy)ethyl-2'-(tributylammonio)ethyl phosphate,
2-((meth)acryloyloxy)propyl-2'-(trimethylammonio)ethyl phosphate,
2-((meth)acryloyloxy)butyl-2'-(trimethylammonio)ethyl phosphate,
2-((meth)acryloyloxy)pentyl-2'-(trimethylammonio)ethyl phosphate,
2-((meth)acryloyloxy)hexyl-2'-(trimethylammonio)ethyl phosphate,
2-(vinyloxy)ethyl-2'-(trimethylammonio)ethyl phosphate,
2-(allyloxy)ethyl-2'-(trimethylammonio)ethyl phosphate,
2-(p-vinylbenzyloxy)ethyl-2'-(trimethylammonio)ethyl phosphate,
2-(p-vinylbenzoyloxy)ethyl-2'-(trimethylammonio)ethyl phosphate,
2-(styryloxy)ethyl-2'-(trimethylammonio)ethyl phosphate,
2-(p-vinylbenzyl)ethyl-2'-(trimethylammonio)ethyl phosphate,
2-(vinyloxycarbonyl)ethyl-2'-(trimethyl ammonio)ethylphosphate,
2-(allyloxycarbonyl)ethyl-2'-(trimethylammonio)ethyl phosphate,
2-(acryloylamino)ethyl-2'-(trimethylammonio)ethyl phosphate,
2-(vinylcarbonylamino)ethyl-2'-(trimethylammonio)ethyl phosphate,
2-(allyloxycarbonylamino)ethyl-2'-(trimethylammonio)ethyl phosphate,
2-(buteroyloxy)ethyl-2'-(trimethylammonio)ethyl phosphate,
2-(crotonoyloxy)ethyl-2'-(trimethylammonio)ethyl phosphate,
ethyl-(2'-trimethylammonioethylphosphorylethyl)fumarate,
butyl-(2'-trimethylammonioethylphosphorylethyl)fumarate,
hydroxyethyl-(2'-trimethylammonioethylphosphorylethyl)fumarate,
ethyl-(2'-trimethylammonioethylphosphorylethyl)fumarate,
butyl-(2'-trimethylammonioethylphosphorylethyl)fumarate, and
hydroxyethyl-(2'-trimethylammonioethylphosphorylethyl)fumarate.

4. The polymer compound of Claim 1, wherein R₂ is independently selected from the group consisting of hydrogen, methyl, ethyl, propyl, butyl, pentyl, hexyl, cyclohexyl, heptyl, octyl, nonyl, decyl, lauryl, tetradecyl, hexadecyl, isobornyl, dicyclopentanyl, dicyclopentenyl, benzyl, 2-methoxyethyl, 2-ethoxyethyl, succinyl, monoalkyl succinate, monoalkyl fumarate, glycidyl, 3,4-epoxybutyl, 2,3-epoxycyclohexyl, 3,4-epoxycyclohexylmethyl , 3-methyloxetane-3-methyl, 3-ethyloxetane-3-methyl, NH₂, NHCH₃, allyl, 2-hydroxyethyl, 3-oxypropyl methyldimethoxysilane, tetrahydrofurfuryl, ethylene glycol dicyclopentenyl ether, 2-ethyl-2-methyl-1,3-dioxoreynylmethyl, 2-isocyanatoethyl, 4-morpholine, phenylglycidylether, phenoxydiethyleneglycol, methoxypolyethyleneglycol, 2-[4-(1-methyl-1-phenylethyl)-phenoxy]ethyl, t-butyl, 2-oxypropyl hexahydrophthalate, gamma-butyrolactone, propoxyethyl, n-isobutoxymethyl, 2-oxy ethylphthalate, 1,1,1,3,3,3-hexafluoroisopropyl, octafluoropentyl, tetrafluoropropyl, heptadecafluorodecyl, and tribromophenyl.

5. The polymer compound of Claim 1, wherein R₃ is independently selected from the group consisting of phenyl, p-methylphenyl, and p-acetoxyphenyl.

6. The polymer compound of Claim 1, wherein R₄ is methylene group or ethylene group.

7. The polymer compound of Claim 1, wherein R₅, R₆ and R₇ is each independently selected from the group consisting of hydrogen, methyl, ethyl, n-propyl, isopropyl, n-butyl, tert-butyl, phenyl, naphthyl, vinyl, methacryloxymethyl, 2-methacryloxyethyl, 3-methacryloxypropyl, acryloxymethyl, 2-acryloxyethyl, 3-acryloxypropyl, 3-glycidyloxypropyl, 2-epoxycyclohexylethyl, 3-epoxycyclohexylpropyl, and O-R₁₂ (where R₁₂ is hydrogen, methyl, ethyl, or propyl).

8. An inorganic nano powder having a surface to which the polymer compound of any one of Claims 1 to 7 is chemically bonded.

9. A powder formulation comprising the inorganic nano powder of Claim 8.

10. The powder formulation of Claim 9, wherein the powder formulation is UV blocking powder, cosmetic powder, or color powder.
